# EUROPEAN PATENT APPLICATION

(11) **EP 4 154 906 A1**
(43) Date of publication of application: **29.03.2023**
(21) Application number: 22197662.4
(22) Date of filing: 26.09.2022
(51) Int. Cl.: A61K 39/00, C07B 63/00

(54) **AN IMPROVED PROCESS FOR QUANTIFICATION OF PURITY IN PROTEIN SOLUTION**

(30) Priority: 24.09.2021 IN 202121043419
(71) Applicant: Kashiv Biosciences, LLC, Piscataway, NJ 08854 (US)
(72) Inventor: SHARMA, Shalini, Piscataway, 08854 (US); KATRODIYA, Hetal, 380058 Gujarat (IN)
(74) Representative: Haseltine Lake Kempner LLP

(57) **Abstract**

The present invention relates to an improved method of quantification and/or estimation of purity of antibody or fusion protein from a protein mixture containing impurity by providing long term sample stability. The invention provides a stable protein solution comprising antibody having reduce formation of Fab and Fc related impurities.

## Description

### Field of the Invention

The present invention relates to an improved method of quantification and/or estimation of purity of antibody or fusion protein from a protein mixture containing impurity by providing long term sample stability. The invention provides a stable protein solution comprising antibody having reduce formation of Fab and Fc related impurities.

### Background of the invention

The development of a monoclonal antibodies (mAbs) candidate into a therapeutic drug is a long, expensive and often unsuccessful process.

Monoclonal antibodies associated with different types of product and process related impurities. However, the product-related impurity isolation and characterisation is an important aspect of biopharmaceutical process validation. It determines the properties of molecular variants that differ in regard to activity, efficacy and safety from the desired drug substance.

The characterization of mAbs becomes difficult because of product and process related impurities. Moreover, one of the major challenges is to get consistent and reproducible analytical result of product which do not have variation and that need to be required to comply with regulatory bodies. Different chromatographic techniques are known for quantification of mAbs but fail to provide consistent result due to the product solution instabilities that make quantification more difficult and lengthier. Product solution instabilities is a major challenge in biopharmaceutical and creates huge burden for skilled person to maintain the stability to avoid any inconsistency in results. If skilled person does not test all protein sample in specified time limit, he has to either discard the protein sample or go with compromised result which creates inconsistency in batch-to-batch similarity.

Hence, there is a need to provide a stable protein solution or sample which gives consistent result. Further, the present invention also provides a stable protein solution by using a protease inhibitor. Invention also provides a quantification method to determine the impurity present in protein sample.

### Summary of the Invention

In an embodiment, the present invention provides a process for quantification of purity of treated protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto a suitable chromatography;
e) eluting the treated protein solution from the suitable chromatography;
wherein the treated protein solution has an improved purity profile in comparison to a digested protein solution prepared without the addition of protease inhibitor.

In an embodiment, the present invention provides a process for the preparation of stable protein solution comprising;
a) protein solution comprising antibody treated with protease enzyme solution to separate Fab and Fc region of the antibody to form a digested protein solution;
b) incubating the digested protein solution at suitable temperature;
c) incubating the treated protein solution for suitable time at suitable temperature;
d) loading the treated solution onto the suitable chromatography;
e) eluting the treated protein solution from the suitable chromatography;
wherein the treated protein solution has an improved stability in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto a Hydrophobic Interaction chromatography (HIC);
e) eluting the treated protein solution from the HIC chromatography;
wherein the treated protein solution has an improved stability in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fab region of antibody in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fc region of antibody in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fab and Fc region of antibody in comparison to a digested protein solution prepared without the addition of protease inhibitor.

In an embodiment, the present invention provides a process for quantification of purity of treated protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity profile in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fab region of antibody in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fc region of antibody in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fab and Fc region of antibody in comparison to a digested protein solution prepared without the addition of protease inhibitor.

In an embodiment, the present invention provides a process for quantification of purity of treated protein solution comprising;
a) protein solution comprising antibody treated with Papain enzyme solution to separate Fab and Fc region of the antibody to form a digested protein solution;
b) incubating the digested protein solution at suitable temperature;
c) treating the digested protein solution with Antipain inhibitor to form a treated protein solution;
d) loading the treated protein solution onto a suitable chromatography;
e) eluting the treated protein solution from the suitable chromatography;
wherein the protein solution has an improved purity profile in comparison to a digested protein solution prepared without the addition of protease inhibitor.

In an embodiment, the present invention provides a process for quantification of purity of treated solution comprising;
a) protein solution comprising antibody treated with Papain enzyme solution to separate Fab and Fc region of the antibody to form a digested protein solution;
b) incubating the digested protein solution for suitable time at suitable temperature;
c) treating the digested protein solution with Antipain inhibitor to form a treated protein solution;
d) loading the treated protein solution onto a Hydrophobic Interaction chromatography (HIC);
e) eluting the treated protein solution from the HIC chromatography;
wherein the treated protein solution has an improved purity of Fab and Fc region of antibody in comparison to a digested protein solution prepared without the addition of protease inhibitor. In another embodiment, the protein solution is treated with protease inhibitor immediately after incubation.

In another embodiment, the protein solution treated with protease inhibitor is stable at least at room temperature for one day, and two days.

In another embodiment, the protein solution treated with protease inhibitor is stable at least at room temperature about 1hour, about 2hours, about 3hours, about 4hours, 5hours, about 6hours, about 7hours, about 8hours, about 9hours, about 10hours, about 11hours, about 12hours, about 13hours, about 14hours, about 15hours, about 16hours, about 17hours, about 18hours, about 19hours, about 20hours, about 21hours, about 22hours, about 23hours, about 24hours, about 25hours, about 26hours, about 27hours, about 28hours, about 29hours, about 30hours, about 31hours, about 32hours, about 33hours, about 34hours, about 35hours, about 36hours, about 37hours, about 38hours, about 39hours, about 40hours, about 41hours, about 42hours, about 43hours, about 44hours, about 45hours, about 46hours, about 47hours, about 48hours, about 49hours, about 50hours, about 51hours, about 52hours, about 53hours, about 54hours, about 55hours, about 56hours, about 57hours, about 58hours, about 59hours, and about 60hours.

In another embodiment, the protein solution treated with protease inhibitor is stable at least at 2⁰C to 8⁰C for at least about 30 minutes, about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days.

### Detail description of the Invention

The present invention relates to an improved method of quantification and/or estimation of purity of antibody or fusion protein from a protein mixture containing impurity by providing long term sample stability. The invention provides a stable protein solution comprising antibody having reduce formation of Fab and Fc related impurities.

As used throughout the specification and in the appended claims, the singular forms "a," "an," and "the" include the plural reference unless the context clearly dictates otherwise.

The term "about", as used herein, is intended to refer to ranges of approximately 10-20% greater than or less than the referenced value. In certain circumstances, one of skill in the art will recognize that, due to the nature of the referenced value, the term "about" can mean more or less than a 10-20% deviation from that value.

The term "one day" or "1 day" used herein refers to 12hours. In certain embodiment "1 day" refers to 24 hours.

The term "comprises" or "comprising" is used in the present description, it does not exclude other elements or steps. For the purpose of the present invention, the term "consisting of" is considered to be an optional embodiment of the term "comprising of". If hereinafter a group is defined to comprise at least a certain number of embodiments, this is also to be understood to disclose a group which optionally consists only of these embodiments.

The term "antibody" includes an immunoglobulin molecule comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region (CH). The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, and FR4.

In certain embodiment, the protein can be antibody or fusion protein. In another embodiment, the antibody or fusion proteins are selected from Etanercept, Rituximab, Palivizumab, Infliximab, Trastuzumab, Alemtuzumab, Adalimumab, Ibritumomab, Omalizumab, Cetuximab, Bevacizumab, Natalizumab, Eculizumab, Certolizumab pegol, Ustekinumab, Canakinumab, Golimumab, Ofatumumab, Tocilizumab, Denosumab, Belimumab, Ipilimumab, Brentuximab, Vedotin, Pertuzumab, Trastuzumab emtansine, Raxibacumab, Obinutuzumab, Siltuximab, Ramucirumab, Vedolizumab, Nivolumab, Pembrolizumab, Darucizumab, Necitumumab, Dinutuximab, Secukinumab, Mepolizumab, Alirocumab, Evolocumab, Daratumumab, Elotuzumab, Ixek izumab, Reslizumab, Aratumab, Bezlotoxumab, Atezolizumab, Obiltoxaximab, Sarilumab, Ocrelizumab ,Tildrakizumab, Romosozumab, Brolucizumab, Crizanlizumab.

Omalizumab (Xolair^{®}) is a recombinant DNA-derived humanized IgGlK monoclonal antibody that selectively binds to human immunoglobulin (IgE). The antibody has a molecular weight of approximately 149 kD. Xolair^{®} is produced by a Chinese hamster ovary cell suspension culture in a nutrient medium containing the antibiotic gentamicin. Gentamicin is not detectable in the final product. Xolair^{®} is a sterile, white, preservative-free, lyophilized powder contained in a single-use vial that is reconstituted with Sterile Water for Injection (SWFI), USP, and administered as a subcutaneous (SC) injection.

The term "Hydrophobic Interaction chromatography" or "HIC" used herein include a liquid chromatography to separate and purify biomolecules by their hydrophobic interaction with the hydrophobic ligands coupled to porous media.

The term "High-performance liquid chromatography" or "HPLC" used herein, formerly referred to as high-pressure liquid chromatography, is a technique in analytical chemistry used to separate, identify, and quantify each component in a mixture.

The term "Protease enzyme" used herein includes enzyme that catalyses (increases reaction rate or "speeds up") proteolysis, the breakdown of proteins into smaller polypeptides or single amino acids. Protease enzyme do this by cleaving the peptide bonds within proteins by hydrolysis, a reaction where water breaks bonds. Proteases are involved in many biological functions, including digestion of ingested proteins, protein catabolism (breakdown of old proteins), and cell signalling. Examples of protease enzymes are Papain (*Carica papaya*)*,* bromelain (*Ananas comosus*)*,* cathepsin K (*liverwort*) and calpain (*Homo sapiens*) more preferably Papain enzyme.

In another embodiment, the "Papain enzyme" used herein cleaves immunoglobulin molecules in the hinge region.

In another embodiment, the "protease enzyme" used herein is Papain enzyme that cleaves immunoglobulin molecules in the hinge region which results in the generation of three ~50kDa fragments; two Fab domains and a Fc domain.

In another embodiment, the "Papain enzyme" used herein cleaves immunoglobulin molecules between amino acids histidine-serine and/or histidine-threonine of the hinge region.

The term "Protease inhibitor" includes the molecules that block the activity of protease. Activity of protease enzymes must be inhibited to prevent protein structure changes that may mask the true state of the solution at the time when the biological experiment is terminated, and the analytical phase starts. Examples of Protease inhibitor are Pepstatin, Leupeptin, Aprotinin, Chymostatin, Phenylmethanesulfonyl fluoride (PMSF), and Antipain, more preferably Antipain protease inhibitor.

The term "Digested protein solution" or "Digested solution" used herein are interchangeable and refers to the protein solution treated with protease enzyme to separate the Fab and Fc region of the antibody to form a digested protein solution. The digested protein solution comprises antibody or fragments thereof, protease enzyme solution, any suitable buffer selected from tris, cysteine and EDTA.

The term "Digestion buffer" used herein refers to solution used for the digestion of protein. Example of buffers that is used for digestion includes sodium salt, organic compound, amino acid and other acid buffer and combination of these.

The pH of digestion buffer is adjusted to pH selected from about pH 7 to about pH 8, preferably pH 7.50 ± 0.05. The pH is adjusted to about pH 7 to about pH 8 using 5N to 10N of Hydrochloride acid, more preferably 6N of Hydrochloride acid.

The term "Treated protein solution" or "Post inhibited solution" used herein refers to the digested protein solution, further treated with protease inhibitor to form the treated protein solution. The treated protein solution comprises antibody or fragments thereof, protease enzyme solution, protease inhibitor, any suitable buffer selected from tris, cysteine and EDTA.

The term "Protein solution" and "Protein sample" used herein is interchangeable respectively in the present invention.

The term used "Stable protein solution" used herein refers to protein solution comprising antibody which remains stable for at least 1 day. In certain embodiment it remains stable for at least at 2⁰C to 8⁰C for at least about 30 minutes, about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days. In an embodiment, the stable protein solution is free of sugar and polyol.

The term "Improved stability" used herein refers to the sample or solution having no further degradation/modifications of the digested sample after treated with protease inhibitor.

In another embodiment, the stable protein solution reduces the formation of undesired impurity selected from "Fab P0", "Fc P1" and "Fc P3".

The term "Gradient buffer" refers to solution used for mixture of mobile phase A and mobile phase B.

The term "Mobile phase" used herein refers to mobile phase A. In certain embodiment, refers to mobile phase B. In certain embodiment, refers to combination of mobile phase A and mobile phase B.

The term "Mobile phase A" are solvents selected from acetonitrile, methanol, and water, preferably water.

The term "Mobile phase B" are solvents selected from acetonitrile, methanol, and water, preferably water.

The term used "Improved purity profile" is term related to improved purity of Fab region and/or Fc region, and combination of Fab and Fc region. In certain embodiment the improved purity profile refers to reduce impurity of Fab region and/or Fc region. In certain embodiment the improved purity profile refers to reduce impurity of Fab and Fc region.

The term "P0" is refer to undesired fragment or impurity of Fab region of the product.

The term "P1" is refer to desired fragment or purity of Fab region of the product.

The term "Fc P4" is refer to desired fragment or purity of Fc region of the product.

The term "Fc P1" and "Fc P3" refers to undesired fragments or impurities of Fc region of the product.

The term "Incubation" used herein refers to condition of protein sample after digesting by protease enzyme and kept or incubated at room temperature or favorable condition. In an embodiment, the favorable incubation temperature is at least 25°C to about 60°C, about 25°C to 40°C, preferably about 37°C. The digested protein solution incubated for at least 30 minutes. In certain embodiment, digested protein solution incubated for about 30 minutes to about 24 hours. In certain embodiment, digested protein solution incubated for about 3 hours to about 10 hours. In preferred embodiment, digested protein solution incubated for about 6 hours.

In an embodiment, the present invention provides a process for quantification of purity of treated protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto a suitable chromatography;
e) eluting the treated protein solution from the suitable chromatography;
wherein the treated protein solution has an improved purity profile in comparison to a digested protein solution prepared without the addition of protease inhibitor.

In an embodiment, the present invention provides a process for the preparation of stable protein solution comprising;
a) protein solution comprising antibody treated with protease enzyme solution to separate Fab and Fc region of the antibody to form a digested protein solution;
b) incubating the digested protein solution at suitable temperature;
c) incubating the treated protein solution for suitable time at suitable temperature;
d) loading the treated solution onto the suitable chromatography;
e) eluting the treated protein solution from the suitable chromatography;
wherein the treated protein solution has an improved stability in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto a Hydrophobic Interaction chromatography (HIC);
e) eluting the treated protein solution from the HIC chromatography;
wherein the treated protein solution has an improved stability in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fab region of antibody in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fc region of antibody in comparison to a digested protein solution prepared without the addition of protease inhibitor.

In an embodiment, the present invention provides a process for preparation of stable protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved stability of Fab and Fc region of antibody in comparison to a digested protein solution prepared without the addition of protease inhibitor.

In an embodiment, the present invention provides a process for quantification of purity of treated protein solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity profile in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fab region of antibody in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fc region of antibody in comparison to a digested protein solution prepared without the addition of protease inhibitor.

In an embodiment, the present invention provides a process for quantification of purity of treated solution comprising;
a) protein solution comprising antibody treated with protease enzyme solution to separate Fab and Fc region of the antibody to form a digested protein solution;
b) incubating the digested protein solution at suitable temperature;
c) treating the digested protein solution with a protease inhibitor to form a treated protein solution;
d) loading the treated protein solution onto the Hydrophobic Interaction chromatography (HIC);
e) eluting the treated protein solution from the HIC chromatography;
wherein the treated protein solution has an improved purity of Fab and Fc region of antibody in comparison to a digested protein solution prepared without the addition of protease inhibitor.

In an embodiment, the present invention provides a process for quantification of purity of treated protein solution comprising;
a) protein solution comprising antibody treated with Papain enzyme solution to separate Fab and Fc region of the antibody to form a digested protein solution;
b) incubating the digested protein solution at suitable temperature;
c) treating the digested protein solution with Antipain inhibitor to form a treated protein solution;
d) loading the treated protein solution onto a suitable chromatography;
e) eluting the treated protein solution from the suitable chromatography;
wherein the protein solution has an improved purity profile in comparison to a digested protein solution prepared without the addition of protease inhibitor.

In an embodiment, the present invention provides a process for quantification of purity of treated solution comprising;
a) protein solution comprising antibody treated with Papain enzyme solution to separate Fab and Fc region of the antibody to form a digested protein solution;
b) incubating the digested protein solution for suitable time at suitable temperature;
c) treating the digested protein solution with Antipain inhibitor to form a treated protein solution;
d) loading the treated protein solution onto a Hydrophobic Interaction chromatography (HIC);
e) eluting the treated protein solution from the HIC chromatography;
wherein the treated protein solution has an improved purity of Fab and Fc region of antibody in comparison to a digested protein solution prepared without the addition of protease inhibitor. In another embodiment, the protein solution is treated with protease inhibitor immediately after incubation.

In another embodiment, the protein solution treated with protease inhibitor is stable at least at room temperature for one day, and two days.

In another embodiment, the protein solution treated with protease inhibitor is stable at least at room temperature about 1hour, about 2hours, about 3hours, about 4hours, 5hours, about 6hours, about 7hours, about 8hours, about 9hours, about 10hours, about 11hours, about 12hours, about 13hours, about 14hours, about 15hours, about 16hours, about 17hours, about 18hours, about 19hours, about 20hours, about 21hours, about 22hours, about 23hours, about 24hours, about 25hours, about 26hours, about 27hours, about 28hours, about 29hours, about 30hours, about 31hours, about 32hours, about 33hours, about 34hours, about 35hours, about 36hours, about 37hours, about 38hours, about 39hours, about 40hours, about 41hours, about 42hours, about 43hours, about 44hours, about 45hours, about 46hours, about 47hours, about 48hours, about 49hours, about 50hours, about 51hours, about 52hours, about 53hours, about 54hours, about 55hours, about 56hours, about 57hours, about 58hours, about 59hours, and about 60hours.

In another embodiment, the protein solution treated with protease inhibitor is stable at least at 2⁰C to 8⁰C for at least about 30 minutes, about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days.

In an embodiment, the digested protein solution comprising antibody and/or fragments thereof, protease enzyme solution, Tris, cysteine and optionally EDTA

In an embodiment, the treated protein solution is incubated for at least for 30 minutes.

In an embodiment, the treated protein solution is incubated from about 1 hours, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, preferably about 4 hours to 8 hours, more preferably about 6 hours.

In certain embodiment, the treated protein solution is incubated at suitable temperature selected from about 25⁰C, about 26⁰C, about 27⁰C, about 28⁰C, about 29⁰C, about 30⁰C, about 31⁰C, about 32⁰C, about 33⁰C, about 34⁰C, about 35⁰C, about 36⁰C, about 37⁰C, about 38⁰C, about 39⁰C, about 40⁰C, about 41⁰C, about 42⁰C, about 43⁰C, about 44⁰C, about 45⁰C, about 46⁰C, about 47⁰C, about 48⁰C, about 49⁰C, about 50⁰C, about 51⁰C, about 52⁰C, about 53⁰C, about 54⁰C, about 55⁰C, about 56⁰C, about 57⁰C, about 58°C, about 59°C, about 60°C.

In an embodiment the treated protein solution is incubated at suitable temperature from 25⁰C to 60⁰C, in preferred embodiment the temperature is 37⁰C.

In an embodiment, the undesired impurity is selected from "Fab P0", "Fc P1" and "Fc P3".

In an embodiment, the process for the preparation of stable protein solution comprising;
a) protein solution comprising antibody treated with protease enzyme solution to separate Fab and Fc region of the antibody to form a digested protein solution;
b) incubating the digested protein solution for suitable time at suitable temperature;
c) treating the digested protein solution with a protease inhibitor to form a treated protein solution;
d) loading the treated protein solution onto a suitable chromatography;
e) eluting the treated protein solution from the suitable chromatography;
wherein the treated protein solution has reduced the formation of undesired impurity Fab P0 in comparison to a digested protein solution prepared without the addition of protease inhibitor.

In an embodiment, the digested protein solution prepared without the addition of protease inhibitor has increased Fab P0 impurity more than one-fold or more, twofold or more, three-fold or more.

In certain embodiment, the digested protein solution prepared without the addition of protease inhibitor has increased Fab P0 impurity more than 100 %, 150%, 200%, 220%, 250% and 300%

In certain embodiment, the digested protein solution prepared without the addition of protease inhibitor has increased Fab P0 impurity more than 100 %, 150%, 200%, 220%, 250% and 300% in time period of about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days.

In certain embodiment, the digested protein solution prepared without the addition of protease inhibitor has increased Fab P0 impurity more than 100 %, 150%, 200%, 220%, 250% and 300% in time period of about one day, about two days, about three days, about four days, about five days, about six days, about seven days, about eight days, about nine days, and about ten days when sample kept at least at 2⁰C to 8⁰C.

In an embodiment, the process for the preparation of stable protein solution comprising;
a) protein solution comprising antibody treated with protease enzyme solution to separate Fab and Fc region of the antibody to form a digested protein solution;
b) incubating the digested protein solution for suitable time at suitable temperature;
c) treating the digested protein solution with a protease inhibitor to form a treated protein solution;
d) loading the treated protein solution onto a suitable chromatography;
e) eluting the treated protein solution from the chromatography;
wherein the treated protein solution has reduced the formation of undesired impurity Fc PI in comparison to a digested protein solution prepared without the addition of protease inhibitor.

In an embodiment, the digested protein solution prepared without the addition of protease inhibitor has increased Fc PI impurity more than one-fold or more, twofold or more, three-fold or more.

In an embodiment, the process for the preparation of stable protein solution comprising;
a) protein solution comprising antibody treated with protease enzyme solution to separate Fab and Fc region of the antibody to form a digested protein solution;
b) incubating the digested protein solution for suitable time at suitable temperature;
c) treating the digested protein solution with a protease inhibitor to form a treated protein solution;
d) loading the treated protein solution onto a suitable chromatography;
e) eluting the treated protein solution from the chromatography;
wherein the treated protein solution has reduced the formation of undesired impurity Fc P3 in comparison to a digested protein solution prepared without the addition of protease inhibitor.

In an embodiment, the digested protein solution prepared without the addition of protease inhibitor has increased Fc P3 impurity more than one-fold or more, twofold or more, three-fold or more.

In another embodiment, the protein solution is treated with protease inhibitor immediately after incubation.

In an embodiment, the stable protein solution eluted from suitable chromatography is quantified by RP-HPLC or UPLC.

In another embodiment, the protein solution treated with protease inhibitor is stable at least at room temperature for one day.

In another embodiment, the protein solution treated with protease inhibitor is stable at least at room temperature for two days.

In another embodiment, the protein solution treated with protease inhibitor is stable at least at room temperature about 1hour, about 2hours, about 3hours, about 4hours, 5hours, about 6hours, about 7hours, about 8hours, about 9hours, about 10hours, about 11hours, about 12hours, about 13hours, about 14hours, about 15hours, about 16hours, about 17hours, about 18hours, about 19hours, about 20hours, about 21hours, about 22hours, about 23hours, about 24hours, about 25hours, about 26hours, about 27hours, about 28hours, about 29hours, about 30hours, about 31hours, about 32hours, about 33hours, about 34hours, about 35hours, about 36hours, about 37hours, about 38hours, about 39hours, about 40hours, about 41hours, about 42hours, about 43hours, about 44hours, about 45hours, about 46hours, about 47hours, about 48hours, about 49hours, about 50hours, about 51hours, about 52hours, about 53hours, about 54hours, about 55hours, about 56hours, about 57hours, about 58hours, about 59hours, and about 60hours.

In another embodiment, the protein solution treated with protease inhibitor is stable at least at 2⁰C to 8⁰C for suitable duration selected from at least about 30 minutes, about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days.

In another embodiment, the protein solution treated with protease inhibitor is stable at least at 2⁰C to 8⁰C for suitable duration selected from about one day, about two days, about three days, about four days, about five days, about six days, and about seven days.

In certain embodiment, the invention provides the use of Hydrophobic Interaction chromatography (HIC), immunoaffinity chromatography, ion exchange chromatography, and reverse phase high performance liquid chromatography (RP-HPLC) for quantification of monoclonal antibody.

In another embodiment said chromatography are suitable for quantification of treated protein solution.

In a preferred embodiment, the invention provides the use of Hydrophobic Interaction chromatography for quantification of monoclonal antibody.

In another embodiment, the column used for HIC is selected from ligand based on Butyl, Methyl, ether, hexyl, octyl, isopropyl, and phenyl ligand-based column.

In a preferred embodiment, column used for HIC are selected from ligand based on phenyl ligand-based column.

In a preferred embodiment, column used for HIC are selected from phenyl ligand-based column TSKgel phenyl column.

In an embodiment, the HIC is performed in bind elute mode which comprises solvent A (mobile phase A) and Solvent B (mobile phase B) at suitable ratio and at suitable pH.

In an embodiment, HIC is performed in bind-elute mode comprises:
a) suitable mobile phase A selected from ammonium chloride, Sodium chloride, Tris, and Ammonium sulphate having concentration selected form 1M to 5M at about pH 7.5 and mobile phase B selected from Sodium Phosphate, Tris- HCl, HEPES, Glycine - NaOH, Tris - Acetate and Tris having concentration from 10mM to about 40mM at about pH 7.5;
b) binding is performed when mobile phase A is substantially present at least more than 95% and/or;
c) elution is performed by decreasing mobile phase A and increasing mobile phase B.

In another embodiment, the salt used in mobile phase A are selected from ammonium chloride, and/or Sodium chloride, Tris, and Ammonium sulphate and pH selected from about 7 to about 8.

In preferred embodiment, the salt used in mobile phase A are Tris, and Ammonium sulphate.

In another embodiment, the salt concentration use in mobile phase A is selected from about 1M to about 5M of Ammonium sulphate.

In another embodiment, the salt concentration use in mobile phase A is selected from about 2M to about 5M of Ammonium sulphate.

In preferred embodiment, the salt concentration use in mobile phase A is about 2M of Ammonium sulphate.

In another embodiment, the salt concentration use in mobile phase A is selected from about 10mM to about 40mM of Tris.

In another embodiment, the salt concentration use in mobile phase A is selected from about 20mM to about 40mM of Tris.

In preferred embodiment, the salt concentration use in mobile phase A is about 20mM of Tris. In another embodiment, the salts use in mobile phase B are selected from Sodium Phosphate, Tris- HCl, HEPES, Glycine - NaOH, Tris - Acetate, Tris and pH selected from about 7 to 8.

In preferred embodiment, the salt use in mobile phase B is Tris.

In another embodiment, the salt concentration use in mobile phase B is selected from about 10mM to about 40mM of Tris.

In another embodiment, the salt concentration use in mobile phase B is selected from about 20mM to about 40mM of Tris.

In preferred embodiment, the salt concentration use in mobile phase B is about 20mM of Tris. In another embodiment, the pH of mobile phase A and mobile phase B is adjusted to pH selected from about pH 7 to about pH 8, about pH 7.3 to about pH 8, about pH 7.5 to about pH 8, and about pH 7.7 to about pH 8.

In another embodiment, the pH of mobile phase A and mobile phase B is adjusted to pH selected from about pH 7 to about pH 8, about pH 7.3 to about pH 8, about pH 7.5 to about pH 8, and about pH 7.7 to about pH 8 using acidic solvent.

In another embodiment, the pH of mobile phase A and mobile phase B is adjusted to pH selected from about pH 7 to about pH 8, about pH 7.3 to about pH 8, about pH 7.5 to about pH 8, and about pH 7.7 to about pH 8 using 5N to 10N of Hydrochloride acid, more preferably 6N of Hydrochloride acid.

In preferred embodiment, the pH of mobile phase A and mobile phase B is adjusted to about pH 7.50 ± 0.05 using acidic solvent.

In preferred embodiment, the pH of mobile phase A and mobile phase B is adjusted to about pH 7.50 ± 0.05 using 5N to 10N of Hydrochloride acid, more preferably 6N of Hydrochloride acid.

In one embodiment, the flow rate of mobile phase is selected from about 0.5 to 1.5 mL/min.

In another embodiment, the flow rate of mobile phase is selected from about 1.0 to 1.5 mL/min.

In preferred embodiment, the flow rate of mobile phase is about 1.0 mL/min.

In one embodiment, the run time of sample through HPLC is selected from about 40 to 80 minutes. In another embodiment, Sample run for about 0 to 5 minutes for binding on column.

In an embodiment, the present invention, the fragments Fab and Fc of monoclonal antibody are analysed through RP-HPLC equipped with a pump, an autosampler, a UV detector and a suitable data acquisition system.

In an embodiment, the mobile phase used in RP-HPLC are mobile phase A and mobile phase B.

In another embodiment, the solvents used in mobile phase A are acetonitrile, methanol, and water.

In certain embodiment, the solvent used in mobile phase A is methanol.

In another embodiment, the solvents used in mobile phase B are acetonitrile, methanol, and water.

In certain embodiment, the solvent used in mobile phase B is acetonitrile.

In an embodiment, the protein is quantified through HIC, where the protein gets bind and elutes from the HIC column.

In certain embodiment, protein binds to column when mobile phase A is decreased. In certain embodiment, protein binds to column when mobile phase A is decreased from about 75% to about 70%. In certain embodiment, protein binds to column when mobile phase A is decreased from about 75% to about 65%. In certain embodiment, protein binds to column when mobile phase A is decreased from about 75% to about 60%.

In certain embodiment, proteins bind to column when mobile phase A is decreased at least by about 5%. In certain embodiment, proteins bind to column when mobile phase A is decreased at least by about 10%. In certain embodiment, proteins bind to column when mobile phase A is decreased at least by about 15%.

In certain embodiment, protein binds to column when mobile phase B is increased. In certain embodiment, protein binds to column when mobile phase B is increased from about 25% to about 30%. In certain embodiment, protein binds to column when mobile phase B is increased from about 25% to about 35%. In certain embodiment, protein binds to column when mobile phase B is increased from about 25% to about 40%.

In certain embodiment, proteins bind to column when mobile phase B is increased at least by about 5%. In certain embodiment, proteins bind to column when mobile phase B is increased at least by about 10%. In certain embodiment, proteins bind to column when mobile phase B is increased at least by about 15%.

In an embodiment, proteins bind to column when mobile phase A is decreased from about 75% to about 60% and mobile phase B increased by about 25% to about 40%.

In certain embodiment, protein elutes from column when mobile phase A is decreased. In certain embodiment, protein elutes from column when mobile phase A is decreased from about 60% to about 50%. In certain embodiment, protein elutes from column when mobile phase A is decreased from about 60% to about 40%. In certain embodiment, protein elutes from column when mobile phase A is decreased from about 60% to about 30%. In certain embodiment, protein elutes from column when mobile phase A is decreased from about 60% to about 20%.

In certain embodiment, protein elutes from column when mobile phase A is decreased at least by about 10%. In certain embodiment, protein elutes from column when mobile phase A is decreased at least by about 20%. In certain embodiment, protein elutes from column when mobile phase A is decreased at least by about 30%. In certain embodiment, protein elutes from column when mobile phase A is decreased at least by about 40%. In certain embodiment, protein elutes from column when mobile phase A is decreased at least by about 50%.

In certain embodiment, protein elutes from column when mobile phase B is increased. In certain embodiment, protein elutes from column when mobile phase B is increased from about 40% to about 50%. In certain embodiment, protein elutes from column when mobile phase B is increased from about 40% to about 60%. In certain embodiment, protein elutes from column when mobile phase B is increased from about 40% to about 70%. In certain embodiment, protein elutes from column when mobile phase B is increased from about 40% to about 80%.

In certain embodiment, protein elutes from column when mobile phase B is increased at least by about 10%. In certain embodiment, protein elutes from column when mobile phase B is increased at least by about 20%. In certain embodiment, protein elutes from column when mobile phase B is increased at least by about 30%. In certain embodiment, protein elutes from column when mobile phase B is increased at least by about 40%.

In an embodiment, protein elutes from column when mobile phase A is decreased from about 60% to about 20% and mobile phase B increased by about 40% to about 80%.

In an embodiment, the washing of HIC column is performed with mobile phase B having the concentration at least about 75%. In certain embodiment, the washing of HIC column is performed with mobile phase B having the concentration at least about 90%. In certain embodiment, the washing of HIC column is performed with mobile phase B having the concentration at least about 100%.

In an embodiment, equilibration is performed with mobile phase A and mobile phase B wherein the mobile phase A and mobile phase B having the ratio of about 3:1.

In preferred embodiment, the run time of sample through HPLC is selected from about 50 to about 60 minutes.

In most preferred embodiment, the run time of sample through HPLC is about 60 minutes.

In an embodiment, the present invention shows by addition of protease inhibitor after incubation there was no significant difference observed in the sample of day 1 post inhibited reference standard solution vs sample of day 7 post inhibited reference standard solution in Table 2.

In an embodiment, the present invention shows the comparative effect on the purity profile wherein the protein solution with protease inhibitor has an improved purity profile in comparison to protein solution prepared without addition of protease inhibitor.

**The present invention provides an example for illustration purpose which should not be considered to limit the scope of the present invention with the described examples.**

### Examples:

### Process for quantification of purity of protein solution

### Materials and reagents:

Use of ACS or HPLC grade materials where applicable, water, ammonium sulfate, EDTA, disodium salt, dihydrate, Tris (2-aminso-2-(hydroxymethyl)-1,3-propanediol), L-cysteine, Papain enzyme from papaya latex, Antipain protease inhibitor, 10N Hydrochloride acid, Xolair^{®} injection, for subcutaneous use. In-house reference standard with known purity. Water (Milli-Q or equivalent).

### Equipment used in present invention are given below:

1. HPLC system equipped with
   a. a pump, an autosampler,
   b. a UV detector and
   c. a suitable data acquisition system.
2. TOSOH Bioscience TSKgel Phenyl-5PW, 7.5 x 75mm, 10µm column
3. Analytical balance
4. Stirrer
5. 10kD Amicon filter
6. pH meter
7. Heating block
8. 0.2 µm PES membrane filter
9. Standard laboratory cleaned glassware
10. Adjustable volume pipetteds and pipetted tips
11. Kim wipes

### Chromatographic Conditions:

| | |
|---|---|
| HPLC system: | HPLC system equipped with a pump, an autosampler, a UV detector and a suitable data acquisition system |
| Column: | TOSOH TSKgel Phenyl- 5PW Column, 10µm, 7.5 x 7.5mm |
| Mobile Phase A: | 2M Ammonium sulfate + 20mM Tris, pH 7.5 |
| Mobile Phase B: | 20mM Tris, pH 7.5 |
| Detection: | UV at 215nm |
| Flow Rate: | 1.0mL/min |
| Injection Volume: | 40µL |
| Injection Amount: | 40µg |
| Column Temp.: | 40°C |
| Sample Temp.: | 4°C |
| Run time: | 60 minutes |
| Needle Wash: | 5% (v/v) Methanol in water |

### PREPARATION OF SOLUTIONS:

### Preparation of papain enzyme stock solution (2mg/ml)

For the preparation of solution accurately weighed and transferred about 2mg of Papain enzyme into a suitable container. Added 1000µL of water to dissolved and mixed well.

### A. Preparation of 1M Tris + 40mM EDTA stock solution:

For the preparation of stock solution weighed and transferred about 12.1g of Tris and 1.50g of EDTA disodium salt, dehydrate into a suitable container. Dissolved in 100mL of water. Adjusted pH to 7.50 ± 0.05 using 6N HCl. The solution was freshly prepared within 24 hours.

### B. Preparation of digestion buffer (100mM Tris + 4mM EDTA + 1mM L-Cysteine solution):

For preparation of digestion buffer accurately measured 10mL of the stock solution prepared in A and transferred into a suitable container. Added 90mL of DI water and mixed well. Weighed and transferred 12mg of L-Cysteine into the container. Dissolved completely. Adjusted pH to 7.50 ± 0.05 using 6N HCL. The solution was freshly prepared within 24 hours.

### C. Preparation of Mobile phase A (2M Ammonium Sulfate + 20mM Tris, pH 7.5):

For the preparation of Mobile Phase A weighed around 2.42g of Tris and 264.3g of Ammonium Sulfate into a suitable container. Dissolved with DI water to a total volume of 1000mL and mixed well. Adjusted pH to 7.50 ± 0.05 using 6N HCl and filtered with 0.2 µ filter membrane. Degassed at room temperature for 10 minutes. The solution can be stored at room temperature for 2 weeks.

### D. Preparation of Mobile phase B (20mM Tris, pH 7.5):

For the preparation of Mobile Phase B weighed around 2.42g of Tris into a suitable container. Dissolved with DI water to a total volume of 1000mL and mixed well. Adjusted pH to 7.50 ± 0.05 using 6N HCl and filtered with 0.2 µ filter membrane. Degassed at room temperature for 10 minutes. The solution can be stored at room temperature for 2 weeks.

### E. Preparation of Antipain Inhibitor Stock Solution (10mM):

For the preparation of solution accurately weighed and transferred about 5mg of Antipain inhibitor into a suitable container. Added 827µL of water to dissolved and mixed well. This solution can be stored at 2-8°C for 1 week or solution can be stored at -20°C for 6 months. Thawed solution cannot be refrozen.

### Preparation of Blank and Sample Solutions for the estimation.

### Blank:

The preparation of blank solution followed by accurately pipetted 596 µL digestion buffer into suitable container. Then accurately pipetted 4 µL DI water and 4.5 µL of 2mg/mL papain solution into the same container and mixed well. Sealed the container and incubated at 37°C using heating block for 6 hours. Then accurately pipetted 4.5 µL of 10mM Antipain inhibitor stock solution into the same container and mixed well. The solution was freshly prepared.

### Reference Standard Solution:

The preparation of reference standard solution followed by accurately pipetted 596 µL digestion buffer into a suitable container. Then accurately pipetted 4 µL of reference standard with 150mg/mL and 4.5 µL of 2mg/mL papain solution into the same container and mixed well. Sealed the container and incubated at 37°C using heating block for 6hours. Then accurately pipetted 4.5µL of 10mM Antipain inhibitor stock solution into the same container and mixed well. The final concentration of the reference standard is about 1mg/mL. The solution was freshly prepared.

### Test Sample Solution:

The preparation of test sample solution followed by accurately pipetted 600 µg sample into a suitable container and make up the volume with digestion buffer. Then accurately pipetted 4.5 µL papain solution into the same container and mixed well. Sealed the container and incubated at 37°C using heating block for 6hours. Then accurately pipetted Antipain inhibitor stock solution into the same container and mixed well. The solution was freshly prepared.

The resulting digest was analysed with a TOSOH TSK gel Phenyl - 5PW column (7.5 x 75 mm), using an HPLC system equipped with a pump, an autosampler, a UV detector and a suitable data acquisition system. The column was equilibrated with 75% of solvent A containing 2 M ammonium sulfate solution, 20 mM TRIS, pH 7.5 and 25% solvent B containing 20 mM TRIS, pH 7.5. The column temperature was 40°C and the flow rate was 1 mL/mins. Following injection of about 40µg of papain digested reference sample, solvent B remained at 25% for 1 minute, followed by a two-step linear gradient from 25% to 40% solvent B in 3 minutes and then from 40% to 80% solvent B in 43 minutes. Solvent B was then increased to 100% for 2 minutes to regenerate the column, followed by reequilibration at 25% solvent B. The column effluent was monitored at 215 nm.

### Example 1

Example 1 describes the comparison between reference standard injected on day 1(control) and reference standard injected on day 7. Table 1 shows comparative results between the reference standard injected on day 1 and the reference standard injected on day 7. A three-fold increase was observed in Fab P0. This data shows the solution instability observed in papain digested samples.

**Table 1: Comparison of Percent Peak Areas of Reference Standard Injected on Day 1 (control) vs. Reference Standard Injected on Day 7.**

| % Peak Area - Reference Standard Day 1 vs. Day 7 | | | | | |
|---|---|---|---|---|---|
| | Fab Peaks | | Fc Peaks | | |
| Inj ection | P0 | P1 | Fc P1 | Fc P3 | Fc P4 |
| Reference Day 1 | 3.51 | 45.52 | 0.65 | 5.41 | 23.95 |
| Reference Day 7 | 10.56 | 38.33 | 0.74 | 6.33 | 25.01 |
| Difference | -7.05 | 7.19 | -0.09 | -0.92 | -1.79 |

It is evident from the above Table 1 that desired Fab fragment P1 reduced from 45.52 to 38.33 on Day 7 and undesired fraction of Fab P0 increased from 3.51 to 10.56 on day 7 which is increased more than 200%. Further it also shows that undesired Fc fragment P1 and P3 increased on day 7.

### Example 2

Example 2 describes the Post inhibited reference standard injected on day 1 vs. post inhibited reference standard injected on day 7. Table 2 shows comparative results between the post inhibited reference standard injected on day 1 and the post inhibited reference standard injected on day 7. A negligible change is observed in Fab P0. This data shows solution stability in papain digested samples with Antipain inhibitor for up to at least 7 days.

**Table 2: Comparison of Percent Peak Areas of Post Inhibited Reference Standard Injected on Day 1 vs. Post Inhibited Reference Standard Injected on Day 7.**

| Post Inhibited Sample Day 1 vs. Day 7 (% Peak Area) | | | | | |
|---|---|---|---|---|---|
| | Fab Peaks | | Fc Peaks | | |
| Injection | P0 | P1 | Fc P1 | Fc P3 | Fc P4 |
| Post Inhibited Day 1 | 3.36 | 45.86 | 0.59 | 5.29 | 23.81 |
| Post Inhibited Day 7 | 3.28 | 46.09 | 0.60 | 5.29 | 23.52 |
| Difference | 0.08 | -0.23 | -0.01 | 0.00 | 0.29 |

It is evident from the above Table 2 that desired Fab fragment P1 increased from 45.86 to 46.09 and undesired fraction of Fab P0 decreased from 3.36 to 3.28 on day 7 which is significantly controlled compared to P0 shown in Table 1. Further it also shows that undesired Fc fragment P1 and P3 is almost same and controlled on day 7 as shown in Table 1.

### Example 3

Example 3 describes Reference standard injected on day 1(control) vs. post inhibited reference standard injected on day 7. Table 3 shows comparative results between the reference standard injected on day 1 only comprised protease enzyme and the post inhibited sample injected on day 7 had protease enzyme and protease enzyme inhibitor. This data shows that addition of papain inhibitor increases solution stability, and 7-day old sample treated with inhibitor is comparable to day 1 sample (without inhibitor).

**Table 3: Comparison of Percent Peak Areas of Reference Standard Injected on Day 1 (control) vs. Post Inhibited Reference Standard Injected on Day 7.**

| % Peak Area - Reference Standard Day 1 vs. Post Inhibited Reference Standard Day 7 | | | | | |
|---|---|---|---|---|---|
| | Fab Peaks | | Fc Peaks | | |
| Injection | P0 | P1 | Fc P1 | Fc P3 | Fc P4 |
| Reference Day 1 | 3.51 | 45.52 | 0.65 | 5.41 | 23.95 |
| Post Inhibited Day 7 | 3.28 | 46.09 | 0.60 | 5.29 | 23.52 |
| Difference | 0.23 | -0.57 | 0.05 | 0.12 | 0.43 |

It is evident from the above Table 3 that desired Fab fragment P1 increased from 45.52 to 46.09 and undesired fraction of Fab P0 decreased from 3.51 to 3.28 on day 7 which is significantly controlled compared to P0 shown in Table 1.

## Claims

1. A process for the preparation of stable protein solution comprising;
a) protein solution comprising antibody treated with protease enzyme solution to separate Fab and Fc region of the antibody to form a digested protein solution;
b) incubating the digested protein solution for suitable temperature;
c) treating the digested protein solution with a protease inhibitor to form a treated protein solution;
d) loading the treated protein solution onto a suitable chromatography;
e) eluting the treated protein solution from the suitable chromatography;
wherein the treated protein solution has an improved stability in comparison to a digested protein solution prepared without the addition of protease inhibitor.

2. The process as claimed in claim 1, wherein the suitable chromatography is selected from Hydrophobic Interaction chromatography (HIC), immunoaffinity chromatography, ion exchange chromatography, UPLC, HPLC and reverse phase high performance liquid chromatography (RP-HPLC) capable to quantify the treated protein solution.

3. The process as claimed in claim 2, wherein the suitable chromatography is Hydrophobic Interaction chromatography (HIC).

4. The process as claimed in claim 3, wherein the HIC column ligand chemistry is selected from butyl, methyl, ether, hexyl, octyl, isopropyl, and phenyl.

5. The process as claimed in claim 4, wherein the HIC is phenyl ligand based TSKgel phenyl column.

6. The process as claimed in claim 3 wherein the HIC is performed in bind-elute mode comprises:
a) suitable mobile phase A selected from ammonium chloride, Sodium chloride, Tris, and ammonium sulphate having concentration selected form 1M to 5M at about pH 7.5 and mobile phase B selected from Sodium Phosphate, Tris- HCl, HEPES, Glycine - NaOH, Tris - Acetate and Tris having concentration from 10mM to about 40mM at about pH 7.5;
b) binding is performed when mobile phase A is substantially present at least more than 95% and/or;
c) elution is performed by decreasing mobile phase A and increasing mobile phase B.

7. The process as claimed in claim 6 wherein the mobile phase B is increased more than about 20%, about 30%, about 40%, about 45%, about 50%, about 55%, about 60%, about 65%, about 70%, about 75% and about 80%.

8. The process as claimed in claim 6, wherein the mobile phase A concentration is selected from about 2M to about 5M at pH.

9. The process as claimed in claim 6, wherein the mobile phase B concentration is selected from about 20mM to about 40mM.

10. The process as claimed in claim 1, wherein the treated protein solution has an improved stability of Fab &/or Fc region of antibody.

11. The process as claimed in claim 1, wherein the Protease inhibitor is selected from Pepstatin, Leupeptin, Aprotinin, Chymostatin, Phenylmethanesulfonyl fluoride (PMSF), and Antipain.

12. The process as claimed in claim 11, wherein the protease inhibitor is Antipain.

13. The process as claimed in claim 1 wherein the digested protein solution comprising antibody or fragments thereof, protease enzyme solution, Tris, cysteine and optionally EDTA.

14. The process as claimed in claim 1, wherein the treated protein solution is incubated for at least 30 min.

15. The process as claimed in claim 1, wherein the treated protein solution is incubated for about 30 min to about 24 hours preferably about 6 hours.

16. The process as claimed in claim 1, wherein the treated protein solution is incubated at suitable temperature from about 25°C to about 60°C.

17. The process as claimed in claim 16, wherein the treated protein solution is incubated at suitable temperature for 37°C.

18. The process as claimed in claim 1, wherein the stable protein solution is stable at least at room temperature for about 1hour, about 2hours, about 3hours, about 4hours, 5hours, about 6hours, about 7hours, about 8hours, about 9hours, about 10hours, about 1 1hours, about 12hours, about 13hours, about 14hours, about 15hours, about 16hours, about 17hours, about 18hours, about 19hours, about 20hours, about 21hours, about 22hours, about 23hours, about 24hours, about 25hours, about 26hours, about 27hours, about 28hours, about 29hours, about 30hours, about 3 1hours, about 32hours, about 33hours, about 34hours, about 35hours, about 36hours, about 37hours, about 38hours, about 39hours, about 40hours, about 41hours, about 42hours, about 43hours, about 44hours, about 45hours, about 46hours, about 47hours, about 48hours, about 49hours, about 50hours, about 51hours, about 52hours, about 53hours, about 54hours, about 55hours, about 56hours, about 57hours, about 58hours, about 59hours, and about 60hours.

19. The process as claimed in claim 1, wherein the stable protein solution is stable at least at 2°C to 8°C for at least about 30 minutes, about 1hour, about 2hours, about 3hours, about 4hours, about 5hours, about 6hours, about 7hours, about 8hours, about 9hours, about 10hours, about 11hours, about 12hours, about one day, about two days, about three days, about four days, about five days, about six days, about seven days, about eight days, about nine days, and about ten days.

20. The process as claimed in claim 1, wherein the improved purity of profile has substantially reduced the formation of undesired impurity.

21. The process as claimed in claim 20, wherein the undesired impurity is selected from "P0", "Fc P1" and "Fc P3".
